# EUROPEAN PATENT APPLICATION

(11) **EP 2 604 293 A1**
(43) Date of publication of application: **19.06.2013**
(21) Application number: 11193535.9
(22) Date of filing: 14.12.2011
(51) Int. Cl.: A61L 2/14, G02B 21/00, H05H 1/00

(54) **A surface cleaning device and a method of cleaning a surface.**

(71) Applicant: Nederlandse Organisatie voor Toegepast -Natuurwetenschappelijk Onderzoek TNO, 2628 VK Delft (NL)
(72) Inventor: Koster, Norbertus Benedictus, 2628 VK Delft (NL); Maas, Diederik Jan, 2628 VK Delft (NL)
(74) Representative: Jansen, Cornelis Marinus

(57) **Abstract**

The invention relates to a vacuum surface cleaning device comprising a gas generation unit, a gas handling unit a plasma generation unit and a sample cleaning unit, wherein the gas generation unit is adapted to generate at least hydrogen and oxygen gases and to supply the said gases into the gas handling unit, wherein the gas handling unit is adapted to retrieve hydrogen and oxygen separately from a gas mixture provided by the gas generation unit, wherein the gas handling unit being further arranged to provide the retrieved gas into the plasma generation unit, wherein the plasma generation unit being adapted to generate a low energetic plasma from the said retrieved gas and to supply radicals and/or ions in the sample cleaning unit and wherein the sample cleaning unit being adapted to expose a sample to the said radicals and/or ions. The invention further relates to a method of cleaning a surface.

## Description

### FIELD OF THE INVENTION

The invention relates to a surface cleaning device.

The invention further relates to a method of cleaning a surface.

### BACKGROUND OF THE INVENTION

In many fields of technology it may be required to clean a surface of an object to a high degree of purity before a suitable subsequent handling. For example, before a suitable manufacturing step a surface of a substrate may be required to be cleaned with high accuracy. Alternatively or additionally, prior to a surface viewing step the surface may be required to be substantially cleaned. Alternatively or additionally, after a surface viewing step the surface may be required to be substantially cleaned due to contamination caused by the viewing step.

Present surface cleaning methods comprising suitable chemically active reactants are in general too aggressive. Known surface cleaning methods based on hydrogen and oxygen gas flows require a presence of voluminous hydrogen and oxygen gas bottles in the cleaning equipment.

It is a disadvantage of the known chemical methods that the cleaned surface may be destroyed by the aggressive reactants. It is a disadvantage of the known methods using voluminous hydrogen and oxygen bottles that this equipment is potentially not safe due to combustion danger of the gases and, therefore, requires particular safety measures in the vicinity of the equipment as well as service from specialized personnel. In addition, the known systems have a disadvantage that due to cleaning methods applied there within, miniaturization is inhibited.

### SUMMARY OF THE INVENTION

It is an object of the invention to provide a surface cleaning device which has superior operational characteristics and is easy and safe in use. In particular, it is an object of the invention to provide a vacuum surface cleaning device.

To this end the device according to the invention comprises a gas generation unit, a gas handling unit a plasma generation unit and a sample cleaning unit, wherein
- the gas generation unit is adapted to generate a gas and to supply the gas into the gas handling unit,
- the gas handling unit being arranged to provide the gas into the plasma generation unit,
- the plasma generation unit being adapted to generate a plasma comprising particles with an energy below 40 eV from the said retrieved gas and to supply radicals and/or ions in the sample cleaning unit
- the sample cleaning unit being adapted to expose a sample to the said radicals and/or ions.

The technical measure of the invention is based on the insight that a superior surface cleaning device may be provided when a number of units are integrated for enabling real-time production of cleaning means. Accordingly, in the device according to the invention suitable amounts of hydrogen and oxygen gases are generated in real-time in the gas generation unit. It will be appreciated, however, that the said units may be implemented as separate modules, or, alternatively, may be integrated in a common housing of the device of the invention. More in particular, it will be appreciated that some units may be integrated with each other for yielding a unit having a plurality of functionalities.

It is found that the method according to the invention is capable of removing adsorbed carbonaceous layers and/or organic molecules or particles from surfaces with enhanced selectivity.

It will be appreciated that the gas generation unit may be adapted to generate a plurality of gases, such as hydrogen, oxygen, chlorine, etc. using electrolysis of a suitable liquid. Water, a spirit, or water and salt may be selected. In case the gas generation unit generates a plurality of gases they can be provided individually or as a mixture into the gas handling unit. The gas handling unit may be adapted to retrieve the necessary gas, for example, hydrogen and oxygen, separately from a gas mixture provided by the gas generation unit and to supply the retrieved gases separately or as a mixture into the plasma generation unit.

The gas generation unit is provided in fluid connection with the gas handling unit and may comprise means, such as selective conduits for conducting pre-selected gases into the gas handling unit. Preferably, the gas generation unit comprises an electrolysis unit for generating a suitable gas, such as, hydrogen, oxygen or chlorine, for example from a liquid solution. In a particular embodiment of the gas generation unit the electrolysis unit comprises a first electrode for generating a flow of hydrogen and a second electrode for generating a flow of oxygen, the said electrodes cooperating with an aqueous solution of a suitable salt. Preferably, each electrode is provided with a dedicated conduit for supplying hydrogen and oxygen respectively towards the plasma generation unit. It will be appreciated that a method of electrolysis is known per se and, therefore, will not be explained in detail. The gas flows obtainable from a suitable electrolysis system may be as large as 5 - 50 sccm.

In a further embodiment of the device according to the invention the gas handling unit comprises one or more switches, one or more gas pressure sensors and/or one or more flow controllers for supplying a pre-selected retrieved gas into the plasma generation unit.

Preferably, the conduits, the switches and the gas pressure sensors and the controllers are used for directing each gas separately or in a predefined mixture in a vacuum reservoir of the plasma generation unit where the gas molecules are converted into radicals and ions by plasma.

In accordance with a still further aspect of the invention the plasma generation unit comprises a resonant cavity for accommodating a gas as is explained with reference to the foregoing and an RF or microwave source for supplying a pre-selected amount of energy towards the resonant cavity for generating the said free radicals and ions.

It is further found that the RF- or microwave induced plasma is advantageous in that it creates relatively more radicals when compared to existing plasma generators. The plasma potential of the RF and microwave generated plasma may have potential below 40 V and practically no sputtering occurs.

It will be appreciated that in the device according to the invention, the resonant cavity may also act as a faraday cage so that no EM fields are emitted outside the plasma formation region. This prohibits damage to vulnerable surfaces due to eddy current heating or damage to electronics due to sparking caused by the microwaves or RF fields. The cavity may also act as a vacuum restriction towards the chamber, permitting the plasma to be formed in a pressure regime of 0.01 - 5 mbar whilst maintaining a high vacuum (< 10⁻⁴ mbar) in the chamber. Power of the plasma source is typically between 10 and 100 W. Gas flows are in the range between 5 and 50 sccm, but may vary with the size of the plasma source envisioned.

Accordingly, it is found that the device of the invention provides a substantial relaxation of logistic aspects applicable to a cleaning unit and may be easily and comfortably used. In addition, the device according to the invention enables switching between different cleaning modes (using radicals only or radicals and ions) in real time.

In a particular embodiment of the device according to the invention the resonant cavity comprises a filter for intercepting the said ions.

In a still further embodiment of the device according to the invention, the sample cleaning unit comprises a control unit for adjusting the operational characteristics of the device.

In particular, the control unit may be arranged for selectively subjecting the sample towards a flow of free radicals and/or ions emanating from the resonant cavity. More preferably, the control unit is arranged to control the plasma generation unit for adjusting at least plasma intensity and a duration of plasma generation.

The method of cleaning a surface according to an aspect of the invention comprises the steps of:
a. generating at least one gas in real time in a generation unit;
b. supplying the said gas into a chamber;
c. generating a low energetic plasma using the said gas in the said chamber for generating free radicals and/or ions from molecules of the said gas;
d. exposing a sample to the said fee radicals and/or ions for cleaning.

It will be appreciated that for the gas either hydrogen and/or oxygen may be used. These gases may be provided by electrolysis of water. Additionally, or alternatively other gases may be used, such as chlorine, which may be provided from electrolysis of dissolved NaCl. In general water may be mixed with NaCl or any other suitable liquid for generating a required gas. Those skilled in the art will readily appreciate which liquids and therein dissolved salts are suitable for gas generation using electrolysis.

These and other aspects of the invention will be discussed with reference to drawings wherein like reference signs correspond to like elements. It will be appreciated that the drawings are presented for illustrative purposes only and may not be used for limiting the scope of the appended claims.

### BRIEF DESCRIPTION OF THE DRAWINGS

Figure 1 presents in a schematic way an embodiment of the cleaning device in cooperation with an imaging device.
Figure 2 presents in a schematic way an embodiment of a gas generation unit of the device according to the invention.
Figure 3 presents in a schematic way an embodiment of a plasma generation unit of the device according to the invention.
Figure 4 presents in a schematic way an embodiment of a cleaning unit of the device according to the invention in cooperation with an imaging device.

### DETAILED DESCRIPTION OF THE DRAWINGS

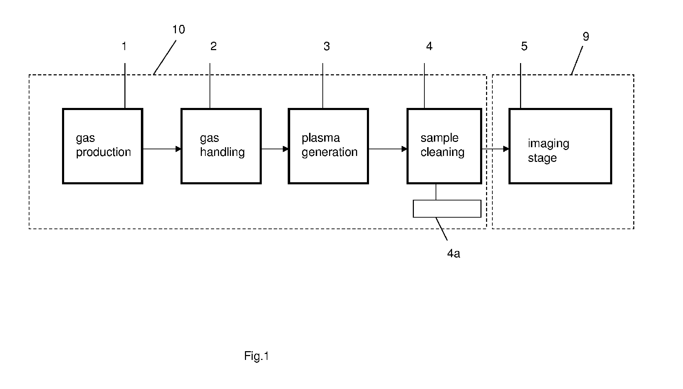

Figure 1 presents in a schematic way an embodiment of the cleaning device in cooperation with an imaging device. In accordance with an aspect of the invention the cleaning device 10 comprises a gas generation unit 1, a gas handling unit 2, a plasma generation unit 3 and a sample cleaning unit 4, wherein the gas generation unit is adapted to generate at least hydrogen and oxygen gases and to supply the said gases into the gas handling unit, the gas handling unit is adapted to retrieve hydrogen and oxygen separately from a gas mixture provided by the gas generation unit, and the gas handling unit is arranged to provide the retrieved gas into the plasma generation unit. The plasma generation unit 3 is arranged to generate low energy plasma, such as plasma below 40 eV, from the said retrieved gas and to supply radicals and/or ions in the sample cleaning unit 4. The sample cleaning unit 4 is adapted to expose a sample to the said radicals and/or ions for surface cleaning purposes.

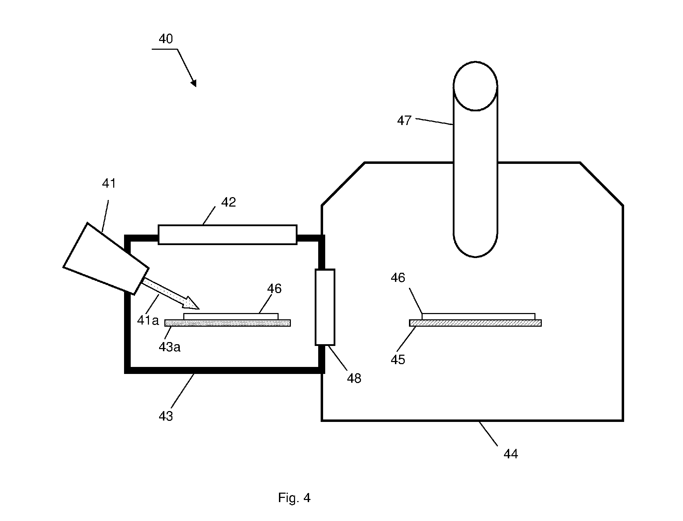

It will be appreciated that preferably, the device 10 is adapted to be operatively connected to a suitable imaging device 9, comprising an imaging stage 5. For example, the device 10 may form part of a microscope so that a platform of the microscope adapted holding a sample is subjected to a stream of free radicals/or ions emanating from the cleaning unit 4 of the device 10. More details on the integrated cleaning and imaging device will be presented with reference to Figure 4. It will be appreciated, however, that any imaging device arranged for sample examination may be used with the system according to the invention.

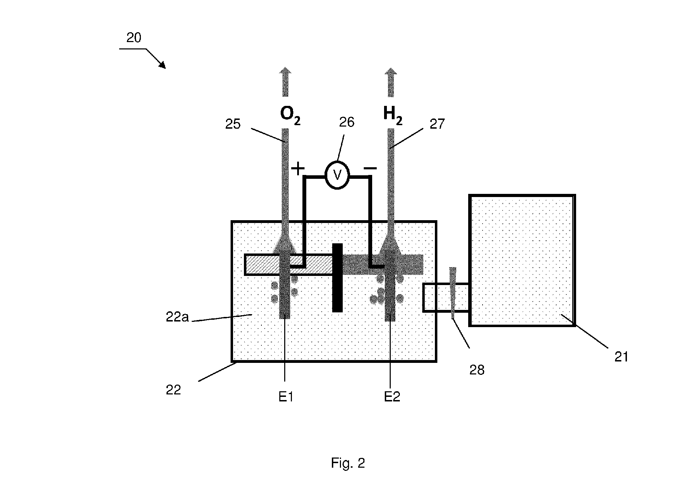

Figure 2 presents in a schematic way an embodiment of a gas generation unit of the device according to the invention. Preferably, the gas generation unit 20 is operating using electrolysis principle. For this purpose a reservoir 22 may be filled, for example, with a suitable mixture 22a of water and, optionally, a salt, which may be provided from a supply reservoir 21 (or alternatively from piping) through a valve 28. In the reservoir 22 the mixture 22a is subjected to a direct current flow from a first electrode E1 to a second electrode E2. The voltage difference between the first electrode E1 and the second electrode E2 is provided by a suitable DC power supply source 26. It will be appreciated that any liquid suitable of producing desired gas by electrolysis may be used. In particular, hydrogen, may be generated by electrolysis of a spirit, such as methanol, ethanol, etc.

Due to the fact that a water solution of salt is an electrolyte, a process of electrolysis shall commence once the power source 26 is electrically connected to the electrodes E1, E2. As a result, oxygen O₂ and hydrogen H₂ shall be accumulated near the electrodes E1, E2.

In the gas generation unit 20 of the device according to the invention the oxygen and hydrogen gases accumulated near the electrodes are collected by means of suitable conduits 25, 27 and are conducted from the gas generation unit to the gas handling unit (not shown). It will be appreciated that theses gases may be collected separately or as a gaseous mixture.

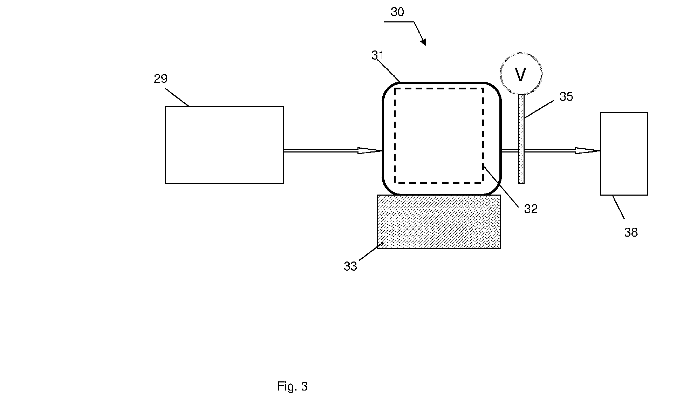

The gas handling unit is adapted to separate oxygen from hydrogen. The gas handling unit may be provided by suitable plurality of valves and switches, pressure meters and flow controllers for suitably selecting a gas from the gaseous mixture and to conducting the selected gas to the plasma generation unit. Other, not used gases from the gaseous mixture provided by the gas generation unit may be collected for a different use or may be exhausted into the atmosphere. Operation of the plasma generation unit will be explained with reference to Figure 3.

It will be appreciated that although in the embodiment of Figure 2 water and salt (NaCl) are used for electrolysis, other liquids are also possible. For example, water alone can be used for generation H₂ and O₂. However, it is found that by adding salt an additional advantage may be obtained. For example it is found possible to create chlorine gas when salt is added to water. Chlorine gas is toxic can be used for Cl* plasma generation.

Figure 3 presents in a schematic way an embodiment of a plasma generation unit of the device according to the invention. The plasma generation unit 30 is arranged to ionize the gases provided by the gas handling unit 29. For this purpose the plasma generation unit 30 comprises a suitable housing 31 comprising a plasma generator 32. It will be appreciated that the gas handling unit may be adapted to select a suitable gas from a gas mixture supplied from the gas generation unit, should the gas generation unit be arranged to generate a plurality of gases. In case when the gas generation unit supplies a single gas, the gas handling unit may be dispensed, or, may be arranged for providing the gas with suitable pressure and flow to the plasma generation unit 32.

Preferably, the plasma generator 32 is provided in a resonant cavity 33 for enhancing plasma for production of free radicals. The plasma generator 32 cooperates with an RF or microwave power delivery system 33 to generate plasma.

In order to enhance plasma generation, the gas handling system 29 may be provided with a controllable flow system for providing a pre-selected flow of a pre-selected gas into the plasma generation unit 30.

Preferably, the plasma generation unit 30 comprises one or more sensors for sensing the plasma quality or output from the plasma generation unit for feeding back to the gas handling unit 29 and/or the power delivery system 33. In this way the device according to the invention may be self-tuning.

Preferably, the plasma generation unit 30 comprises a filter, such as an electrostatic filter 35, for preventing ions of the plasma to enter the sample cleaning unit 38. The electrostatic filter 35 may be provided with a positive bias.

The sample cleaning unit 38 is preferably adapted to selectively remove hydrocarbon molecules from a sample surface by exposing the sample to either only the plasma-generated radicals or the radicals and ions emanating from the plasma generator 30.

More preferably, a tunable amount of radicals, such as H* or O* may be supplied to the surface of a sample which is subjected to cleaning. A user interface (not shown) may be provided for enabling real-time adaptation of the cleaning substance which is impinging the sample. For example, plasma intensity and duration may be adapted in real-time. In general, it is sufficient to carry out user-controlled adaptation of the plasma parameters. However, it is also possible that the plasma parameters are adapted on-line by a control unit (not shown) of the cleaning unit 38. In this case, the control unit may have a feed-back line to the controls of the gas handling unit and/or plasma generation and power supply units.

Figure 4 presents in a schematic way an embodiment of a cleaning unit of the device according to the invention in cooperation with an imaging device. In this particular embodiment the cleaning device is partially integrated (by means of the cleaning unit 43) with an imaging device, such as a microscope 44.

For the sake of simplicity only the cleaning unit 43 of the device according to the invention is shown. The cleaning unit 43 may be arranged to receive radicals and/or ions via a supply port 41 from a plasma generation unit (not shown). For ease of operation handling, the cleaning unit 43 may be provided with a displaceable sample support stage 43a for supporting the sample 46 to a flow 41a of radicals and/or ions emanating from a suitable conduit 41 of the plasma generation unit (not shown). The sample 46 may be provided on the displaceable sample support stage 43a via a door 42. The cleaning unit 43 is connected to the imaging unit 44 by an (ultra) high vacuum door 48, which may be remotely operated.

After the sample 46 has been cleaned, for example, under suitable (ultra) high vacuum conditions, the sample support stage enters the imaging device 44 via the door 48. The cleaned sample 46 may be investigated using a suitable optical column 47. It will be appreciated that the invention prevents against contamination by cracking hydrocarbons with the particles that are used to scan the surface. The cracking occurs when those particles have sufficient energy, which is likely to occur for UV, DUV and EUV photons, electron beams, ion beams, especially when the particle energy exceed about 5 eV.

It will be appreciated that although in the present embodiment the cleaning unit 43 is adjacent the imaging device 44, it is also possible that the cleaning unit 44 forms part of the imaging device, for example, when the conduit 41 is arranged to provide a flow of radicals and/or ions on the sample support stage 45 of the imaging device 44.

While specific embodiments have been described above, it will be appreciated that the invention may be practiced otherwise than as described. Moreover, specific items discussed with reference to any of the isolated drawings may freely be inter-changed supplementing each outer in any particular way. The descriptions above are intended to be illustrative, not limiting. Thus, it will be apparent to one skilled in the art that modifications may be made to the invention as described in the foregoing without departing from the scope of the claims set out below.

## Claims

1. A surface cleaning device comprising a gas generation unit, a gas handling unit, a plasma generation unit and a sample cleaning unit, wherein
- the gas generation unit is adapted to generate a gas and to supply the gas into the gas handling unit,
- the gas handling unit being arranged to provide the gas into the plasma generation unit,
- the plasma generation unit being adapted to generate plasma comprising particles with an energy below 40 eV from the said gas and to supply radicals and/or ions in the sample cleaning unit;
- the sample cleaning unit being adapted to expose a sample to the said radicals and/or ions.

2. The device according to claim 1, wherein the gas generation unit comprises an electrolysis unit for generating any of hydrogen, oxygen and chlorine gases from a liquid solution.

3. The device according to claim 2, wherein the liquid solution comprises water, a spirit or a mixture of water and NaCl.

4. The device according to claim 2 or 3, wherein the gas generation unit is arranged to generate a mixture of gases, the gas handling unit being arranged to select a gas for supply into the plasma generation unit.

5. The device according to claim 1, 2 , 3 or 4, wherein the gas handling unit comprises one or more switches, one or more gas pressure sensors and/or one or more flow controllers for supplying a pre-selected gas into the plasma generation unit.

6. The device according to any one of the preceding claims , wherein the plasma generation unit comprises a resonant cavity for accommodating a gas and an RF or microwave source for supplying a pre-selected amount of energy towards the resonant cavity comprising the gas for generating free radicals and ions.

7. The device according to claim 6, the resonant cavity comprises a filter for intercepting the said ions.

8. The device according to any one of the preceding claims, wherein the sample cleaning unit comprises a control unit for adjusting the operational characteristics of the device.

9. The device according to claim 8, wherein the control unit is arranged for selectively subjecting the sample towards a flow of free radicals and/or ions emanating from the resonant cavity.

10. The device according to claim 8, wherein the control unit is arranged to control the plasma generation unit for adjusting at least plasma intensity and a duration of plasma generation.

11. The device according to any one of the preceding claims, wherein the device is portable.

12. A microscope comprising the device according to any one of the preceding claims.

13. A method of cleaning a surface comprising the steps of:
e. generating a gas in real time in a generation unit;
f. supplying the said gas into a chamber;
g. generating a low energetic plasma using the said gas in the said chamber for generating free radicals and/or ions from molecules of the said gas;
h. exposing a sample to the said fee radicals and/or ions for cleaning.

14. The method according to claim 13, wherein hydrogen, oxygen or chlorine is generated.

15. The method according to claim 13 or 14, wherein the device according to any one of the preceding claims 1 - 12 is used.
